# EUROPÄISCHE PATENTSCHRIFT

(11) **EP 1 458 481 B1**
(45) Veröffentlichungstag und Bekanntmachung des Hinweises auf die Patenterteilung: **05.07.2006**
(21) Anmeldenummer: 02804879.1
(22) Anmeldetag: 06.12.2002
(51) Int. Cl.: B01J 37/03, B01J 23/72, B01J 23/745, C07C 51/295, C07C 51/097, C07C 227/02

(54) **Verfahren ZUR HERSTELLUNG VON CARBONSÄURESALZEN**
Processs FOR PRODUCING CARBOXYLIC ACID SALTS
Procédé DESTINE A LA FABRICATION DE SELS D'ACIDES CARBOXYLIQUES

(30) Priorität: 14.12.2001 DE 10161674
(43) Veröffentlichungstag der Anmeldung: 22.09.2004
(73) Patentinhaber: BASF Aktiengesellschaft, 67056 Ludwigshafen (DE)
(72) Erfinder: WESSEL, Helge, 68167 Mannheim (DE); SEITZ, Verena, 67061 Ludwigshafen (DE); HARTH, Klaus, 67317 Altleiningen (DE); BOMM, Volker, 31675 Bückeburg (DE); AUST, Nicola, Christiane, 68199 Mannheim (DE)
(86) Internationale Anmeldenummer: PCT/EP2002/013842
(87) Internationale Veröffentlichungsnummer: WO 2003/051513

(56) Entgegenhaltungen:
- WO-A-01/77054
- WO-A-94/24091
- US-A- 3 696 152
- US-A- 4 250 111
- US-A- 4 782 183

## Beschreibung

Die vorliegende Erfindung betrifft ein Verfahren zur Herstellung von Carbonsäuresalzen auf der Basis von Kupferkatalysatoren.

Aus der WO-A1-94/24091 sind Katalysatoren für die Herstellung von Aminocarbonsäuresalzen bekannt, die durch Coprecipitation von Kupfer- und Zirkonsalzen mit Chrom-, Titan-, Niob-, Tantal-, Vanadium-, Molybdän-, Magnesium-, Wolfram-, Cobalt-, Nickel-, Wismut-, Zinn-, Antimon-, Blei- und Germaniumsalzen hergestellt wurden.

US-A-4 782 183 beschreibt ein Verfahren zur Herstellung von Carbonsäuren in Gegenwart von Kupferkatalysatoren, wie Raney-Kupfer, Kupfer prezipitiert auf einem Zirkoniumoxid-Träger oder Kupfer, hergestellt durch thermische Zersetzung von Kupferformiat. Der Katalysator enthält kein Eisen.

Diese Katalysatoren lassen jedoch zu wünschen übrig.

Der vorliegenden Erfindung lag daher die Aufgabe zugrunde, ein verbessertes Verfahren für die Herstellung von Carbonsäuresalzen bereitzustellen.

Demgemäß wurden ein verbessertes Verfahren zur Herstellung von Carbonsäuresalzen gefunden, das dadurch gekennzeichnet ist, dass man Alkohole der allgemeinen Formel I

R-CH₂CH₂OH (I),

in der
- R: -(OCH₂CH₂OH oder -NR¹R² und
- R¹, R²: unabhängig voneinander Wasserstoff, Phenyl, -CH₂CH₂OH, C₁- bis C₂₀-Alkyl oder C₂- bis C₅-Dialkylamino
bedeuten, mit Alkalihydroxid, Erdalkalihydroxid oder deren Gemischen in Wasser in Gegenwart eines Kupferkatalysators, der
a) aus Kupfer besteht oder
b) 99,9 bis 10 Gew.-% Kupfer und 0,01 bis 90 Gew.-% Eisen und 0 bis 50 Gew.-% eines oder mehrerer anderer Metalle enthält,
der gegebenenfalls dotiert ist, hergestellt durch Prezipitation von Kupfersalzlösungen bzw. von Kupfer- und Eisensalzlösungen ggf. mit Salzen anderer Metalle durch Coprezipitation mit einer Base und Reduktion mit Wasserstoff,
bei Temperaturen von 120 bis 280°C und einem Druck zwischen dem Dampfdruck des Wassers und 75 bar umsetzt.

Die erfindungsgemäß eingesetzten Katalysatoren kann man wie folgt erhalten:

Man kann diskontinuierlich oder kontinuierlich eine Kupfersalzlösung in einer Prezipitation (Fällung) oder eine Mischung aus einer Kupfersalz- und Eisensalzlösung und ggf. Salzlösungen anderer Metalle in einer Coprezipitation (gemeinsame bzw. simultane Fällung) mit einer Base im Bereich von pH 7 bis 14 bei Temperaturen von 5 bis 100°C, bevorzugt 15 bis 90°C, besonders bevorzugt 20 bis 85°C und einem Druck von 0,1 bis 5 bar, bevorzugt Atmosphärendruck (Normaldruck) umsetzen, das Fällprodukt, in der Regel die entsprechenden Hydroxide, beispielsweise mit Wasser waschen, bei 50 bis 250°C trocknen, gegebenenfalls bei 300 bis 700°C calcinieren und anschließend bei 150 bis 300°C in einem Wasserstoffstrom reduzieren. Die Reduktion kann jedoch auch vor der Calcinierung durchgeführt und ggf. nochmals calciniert werden.

Die Kupfer- bzw. Kupfer-/Eisenkatalysatoren können nach der Coprezipitation oder nach der anschließenden Trocknung oder nach der Calcinierung durch Tränken, stromloses Abscheiden, elektrochemisches Abscheiden, CVD (chemical vapor deposition) oder Sputtern, bevorzugt Tränken oder stromloses Abscheiden, bevorzugt Tränken dotiert werden. Zur Dotierung eignen sich die als "andere Metalle" bezeichneten Elemente, die in Form löslicher Salze oder als Metalle selbst aufgebracht werden können. Werden Salze aufgebracht, wird in der Regel zur Fixierung nachcalciniert. Die Menge an Dotierung kann in weiten Grenzen variieren, liegt aber in der Regel bei 0,001 bis 5 Gew.-%, bevorzugt 0,005 bis 3 Gew.-%, besonders bevorzugt 0,01 bis 2 Gew.-%, insbesonders 0,02 bis 1 Gew.-%.

Als Basen eignen sich beispielsweise Alkalihydroxid, Erdalkalihydroxid, Alkalicarbonat, Erdalkalicarbonat, Alkalihydrogencarbonat, Erdalkalihydrogencarbonat, Ammoniak, wasserlösliche Amine oder deren Gemische. Als Alkalihydroxide eignen sich Lithiumhydroxid, Natriumhydroxid, Kaliumhydroxid, Rubidiumhydroxid und Cäsiumhydroxid, bevorzugt Lithiumhydroxid, Natriumhydroxid und Kaliumhydroxid, besonders bevorzugt Natriumhydroxid und Kaliumhydroxid. Als Erdalkalihydroxide eignen sich Berylliumhydroxid, Magnesiumhydroxid, Calciumhydroxid, Strontiumhydroxid und Bariumhydroxid, bevorzugt Magnesiumhydroxid, Calciumhydroxid, besonders bevorzugt Calciumhydroxid. Als Alkalicarbonate eignen sich Lithiumcarbonat, Natriumcarbonat, Kaliumcarbonat, Rubidiumcarbonat und Cäsiumcarbonat, bevorzugt Lithiumcarbonat, Natriumcarbonat und Kaliumcarbonat, besonders bevorzugt Natriumcarbonat und Kaliumcarbonat. Als Erdalkalicarbonate eignen sich Berylliumcarbonat, Magnesiumcarbonat, Calciumcarbonat, Strontiumcarbonat und Bariumcarbonat, bevorzugt Magnesiumcarbonat, Calciumcarbonat, besonders bevorzugt Calciumcarbonat. Als Alkalihydrogencarbonate eignen sich Lithiumhydrogencarbonat, Natriumhydrogencarbonat, Kaliumhydrogencarbonat, Rubidiumhydrogencarbonat und Cäsiumhydrogencarbonat, bevorzugt Lithiumhydrogencarbonat, Natriumhydrogencarbonat und Kaliumhydrogencarbonat, besonders bevorzugt Natriumhydrogencarbonat und Kaliumhydrogencarbonat. Als Erdalkalihydrogencarbonate eignen sich Berylliumhydrogencarbonat, Magnesiumhydrogencarbonat, Calciumhydrogencarbonat, Strontiumhydrogencarbonat und Bariumhydrogencarbonat, bevorzugt Magnesiumhydrogencarbonat, Calciumhydrogencarbonat, besonders bevorzugt Calciumhydrogencarbonat. Als wasserlösliche Amine eignen sich beispielsweise Ammoniak, Methylamin, Dimethylamin, Trimethylamin, Ethylamin, Diethylamin und Triethylamin, bevorzugt Ammoniak, Dimethylamin, Trimethylamin und Triethylamin, besonders bevorzugt Ammoniak und Trimethylamin.

Als andere Metalle eignen sich alle Metalle der Gruppe IIa, IIIa, IVa, Va, VIa, IIb, IIIb, IVb, Vb, VIb, VIIb und VIII des Periodensystems der Elemente wie Beryllium, Magnesium, Calcium, Strontium, Barium, Bor, Gallium, Indium, Thallium, Germanium, Zinn, Blei, Antimon, Wismut, Selen, Tellur, Silber, Gold, Zink, Cadmium, Scandium, Yttrium, Lanthan, Titan, Hafnium, Vanadin, Niob, Tantal, Chrom, Molybdän, Wolfram, Mangan, Technetium, Rhenium, Cobalt, Nickel, Ruthenium, Rhodium, Palladium, Osmium, Iridium und Platin sowie Cer, bevorzugt Nickel, Cobalt, Chrom, Zink, Lanthan, Ruthenium, Rhodium, Palladium, Iridium und Platin, besonders bevorzugt Nickel, Cobalt, Zink und Lanthan.

Als Salze von Kupfer, Eisen und der anderen Metalle eignen sich in der Regel alle bevorzugt wasserlöslichen anorganischen und organischen Salze.

Als Kupfersalze eignen sich in der Regel alle bevorzugt wasserlöslichen anorganischen und organischen Salze, beispielsweise Kupfernitrat, Kupferchlorid, Kupfersulfat, Kupfercarbonat, Kupferhydrogencarbonat, Kupferhydrogensulfat, Kupferacetylacetonat oder Kupferacetat, bevorzugt Kupfernitrat, Kupferchlorid, Kupfercarbonat oder Kupferacetat, besonders bevorzugt Kupfernitrat, Kupferchlorid oder Kupferacetat.

Als Eisensalze eignen sich in der Regel alle bevorzugt wasserlöslichen anorganischen und organischen Salze, beispielsweise Eisennitrat, Eisenchlorid, Eisensulfat, Eisenhexacyanoferrat, Eisencarbonat, Eisenhydrogencarbonat, Eisenhydrogensulfat, Eisenacetylacetonat oder Eisenacetat, bevorzugt Eisennitrat, Eisenchlorid oder Eisenacetat, besonders bevorzugt Eisennitrat oder Eisenchlorid.

Als Salze anderer Metalle eignen sich in der Regel alle bevorzugt wasserlöslichen anorganischen und organischen Salze, beispielsweise Nitrate, Halogenide, Sulfate, Cyanide, Hydroxide, Carbonate, Hydrogencarbonate, Hydrogensulfate, Acetylacetonate oder Acetate, bevorzugt Nitrate, Halogenide, Sulfate, Carbonate, Hydrogencarbonate oder Acetate, besonders bevorzugt Nitrate, Halogenide, Carbonate oder Acetate.

Die Kupferkatalysatoren enthalten je nach Herkunft des Ausgangsmaterials ggf. Verunreinigungen. Eine Aufreinigung erfolgt in der Regel nicht.

Die Kupfer-/Eisenkatalysatoren enthalten 99,9 bis 10 Gew.-%, bevorzugt 95 bis 20 Gew.-%, besonders bevorzugt 90 bis 30 Gew.-% Kupfer, 0,01 bis 90 Gew.-%, bevorzugt 3 bis 75 Gew.-%, besonders bevorzugt 5 bis 60 Gew.-% Eisen und 0 bis 50 Gew.-%, bevorzugt 0,1 bis 35 Gew.-%, besonders bevorzugt 0,5 bis 25 Gew.-% eines oder mehrere andere Metalle, also ein bis acht, bevorzugt ein bis fünf, besonders bevorzugt ein bis drei, insbesondere ein oder zwei andere Metalle.

Besonders bevorzugt sind solche Katalysatoren, die neben Kupfer 1 bis 90 Gew.-% Eisen enthalten und mit NaOH bei pH 10 bis 11,5 und bei einer Temperatur von 50 bis 80°C gefällt, bei 180 bis 220°C getrocknet, bei 450 bis 550°C calciniert und bei 200 bis 300°C mit Wasserstoff reduziert wurden. Nach der Reduktion mit Wasserstoff enthielten diese Katalysatoren einen Kupferoxidgehalt und/oder Cupritgehalt von 0 bis 35 Gew.-%. Eine besondere Ausführungsform besteht darin, daß der Katalysator einen Aluminiumgehalt von weniger als 4 Gew.-%, bevorzugt zwischen 3,99 und 0 Gew.-%, besonders bevorzugt zwischen 3,99 und 1 Gew.-%, insbesondere zwischen 3,99 und 2 Gew.-%. Eine weitere besondere Ausführungsform besteht darin, daß die Katalysatorherstellung ohne Zirkon erfolgt.

Besonders geeignet für die Herstellung von Carbonsäuresalzen aus Alkoholen haben sich solche erfindungsgemäß eingesetzten Katalysatoren erwiesen, die vor der Reduktion mit Wasserstoff eine CuO-Kristallitgröße von 1 bis 75 nm, besonders bevorzugt 2 bis 50 nm aufweisen.

Das erfindungsgemäße Verfahren zur Herstellung von Carbonsäuresalzen aus Alkoholen kann man wie folgt durchführen:

Man kann in einem Druckbehälter, z.B. einem Autoklaven, einem Rohrreaktor, Umlaufreaktor oder einer Rührkesselkaskade in jeder beliebigen Reihenfolge Alkohol, Base, Wasser und Katalysator einfüllen. Die Base wird bevorzugt vor dem Einfüllen in Wasser gelöst. Bevorzugt legt man den Katalysator vor und fügt den Alkohol getrennt von der wäßrigen Base, oder den Alkohol gemeinsam mit der wäßrigen Base zu. Der verschlossene Druckbehälter wird auf eine Temperatur von 120 bis 280°C, bevorzugt 140 bis 240°C, besonders bevorzugt 150 bis 220°C aufgeheizt, wobei sich ein Eigendruck des Systems aufbaut, der gegebenenfalls erhöht wird. Die Umsetzung erfolgt bei einem Druck zwischen dem Dampfdruck des Wassers und 75 bar, bevorzugt 2 bis 50 bar, besonders bevorzugt 3 bis 30 bar, insbesondere 4 bis 15 bar. In einer besonderen Ausführungsform des Herstellverfahrens enthält die Reaktionsmischung weniger als 4000 ppm, bevorzugt zwischen 3999 und 0 ppm, besonders bevorzugt zwischen 3999 und 1000 ppm, insbesondere zwischen 3999 und 2000 ppm Aluminiumionen.

Als Alkohole eignen sich unter anderen Ethylenglykol(derivate) wie Ethylenglykololigomere oder polymere Ethylenglykole und Aminoalkohole wie Ethanolamin, Diethanolamin, Triethanolamin, N,N-Bis(2-hydroxyethyl)isopropylamin oder Tetrakis(hydroxyethyl)1,2-propylendiamin. Insbesondere eignen sich Alkohole der allgemeinen Formel R-CH₂CH₂OH (I).

Die Reste R, R¹ und R² in den Alkoholen der allgemeinen Formel (I) haben folgende Bedeutung:
- R: -(OCH₂CH₂OH) oder -NR¹R², bevorzugt -NR¹R²,
- R¹, R²: unabhängig voneinander
- Wasserstoff,
- Phenyl,
- -CH₂CH₂OH,
- C₁- bis C₂₀-Alkyl, bevorzugt C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl, insbesonders Methyl und Ethyl,
- C₂- bis C₅-Dialkylamino wie Dimethylamin, Ethylmethylamin, Diethylamin, Ethyl-n-propylamin, Ethyl-iso-propylamin, Methyl-n-propylamin, Methyl-iso-propylamin, Methyl-n-butylamin, Methyl-iso-butylamin, Methyl-sec.-butylamin und Methyl-tert.-butylamin, bevorzugt Dimethylamin, Ethylmethylamin, Diethylamin, Ethyl-n-propylamin, Ethyl-iso-propylamin, Methyl-n-propylamin und Methyl-iso-propylamin, besonders bevorzugt Dimethylamin, Ethylmethylamin, Diethylamin und Ethyl-n-propylamin.

Aminocarbonsäuren bzw. ihre Salze (I) sind wertvolle Zwischenprodukte für Chelatbildner z.B. in Detergentien, für Pharmazeutika, Agrarprodukte wie Pestizide sowie Lebensmittel- und Futterzusatzstoffe. Oxycarbonsäuren bzw. ihre Salze (I), hergestellt aus Ethylenglykol(derivaten) sind wertvolle Zwischenprodukte für z.B. Detergentien.

### Beispiele

### Beispiel 1

2,5 Liter einer wäßrigen Lösung aus 593 g Natriumcarbonat wurde bei 70°C kontinuierlich innerhalb von 20 Min. unter Rühren in 2,5 Liter einer wäßrigen Lösung aus 1536,3 g Kupfernitrat bei einem pH von 7 (unter Zugabe von HNO₃) eingetragen. Nach 30 minütigen Nachrühren bei 70°C, Abfiltrieren, Waschen mit insgesamt 100 Liter Wasser, Trocknung 4 h bei 200°C wurde 4 h bei 230°C mit Wasserstoff reduziert.

Der erhaltene Katalysator wies einen Kupfergehalt von 96,5 Gew.-% und einen Cupritgehalt von 3,5 Gew.-% auf. Die CuO-Kristallitgröße vor der Reduktion mit Wasserstoff betrug 25 nm.

### Beispiel 2

4 Liter einer wäßrigen Lösung aus 948,8 g Natriumcarbonat wurde bei 70°C kontinuierlich innerhalb von 35 Min. unter Rühren in 2,5 Liter einer wäßrigen Lösung aus 1536,3 g Kupfernitrat bei einem pH von 9 (unter Zugabe von HNO₃) eingetragen. Nach 30 minütigen Nachrühren bei 70°C, Abfiltrieren, Waschen mit insgesamt 100 Liter Wasser, Trocknung 4 h bei 200°C wurde 4 h bei 230°C mit Wasserstoff reduziert.

Der erhaltene Katalysator wies einen Kupfergehalt von 97 Gew.-% und einen Cupritgehalt von 3 Gew.-% auf. Die CuO-Kristallitgröße vor der Reduktion mit Wasserstoff betrug 28 nm.

### Beispiel 3

2 Liter einer wäßrigen Lösung aus 660 g Natriumhydroxid wurde bei 70°C kontinuierlich innerhalb von 20 Min. unter Rühren in 2,5 Liter einer wäßrigen Lösung aus 1536,3 g Kupfernitrat bei einem pH von 11 (unter Zugabe von HNO₃) eingetragen. Nach 30 minütigen Nachrühren bei 70°C, Abfiltrieren, Waschen mit insgesamt 100 Liter Wasser, Trocknung 4 h bei 200°C wurde 4 h bei 230°C mit Wasserstoff reduziert.

Der erhaltene Katalysator wies einen Kupfergehalt von 92 Gew.-% und einen Cupritgehalt von 8 Gew.-% auf. Die CuO-Kristallitgröße vor der Reduktion mit Wasserstoff betrug 21 nm.

### Beispiel 4 (nicht erfindungsgemäß)

Analog Beispiel 3 wurde ein Fällgut hergestellt, abfiltriert und gewaschen. Anschließend wurde das Fällgut 4 h bei 150°C getrocknet.

50 g des getrockneten Fällgutes wurden entsprechend seiner Wasseraufnahme (1,00 ml/g) mit 50 ml einer wäßrigen Lösung von 8,95 g Eisen-(III)-nitrat-hydrat innerhalb von 1 h getränkt. Anschließend wurde 4 h bei 120°C getrocknet, 2 h bei 500°C calciniert und 4 h bei 230°C mit Wasserstoff reduziert.

Der erhaltene Katalysator wies einen Eisengehalt von 3 Gew.-% auf. Die CuO-Kristallitgröße vor der Reduktion mit Wasserstoff betrug 20 nm.

### Beispiel 5

2274 g einer 40 Gew.-%igen wäßrigen Lösung von Natriumhydroxid wurde bei 70°C kontinuierlich innerhalb 20 min unter Rühren in 2,5 Liter einer wäßrigen Lösung aus 1238,7 g Kupfernitrat und 357,9 g Eisen-(III)-nitrat-nonahydrat eingetragen. Nach 30 minütigem Nachrühren bei 70°C, Abfiltrieren, Waschen mit 100 Liter Wasser, Trocknung 4 h bei 200°C wurde 4 h bei 230°C mit Wasserstoff reduziert.

Der erhaltene Katalysator wies einen Kupfergehalt von 72 Gew.-% auf. Die CuO-Kristallitgröße vor der Reduktion mit Wasserstoff betrug 9 nm.

### Vergleichsbeispiel A

Analog Beispiel 19 aus der WO-A-94/24091 wurde ein Katalysator hergestellt.

### Durchführung der Dehydrierungen

Ein Autoklav mit einem Innenvolumen von 300 ml wurde mit 40 g Diethanolamin, 32 g Natriumhydroxid, 85 g Wasser und 4 g des jeweiligen Katalysators beschickt. Die Umsetzung erfolgte bei einer Temperatur von 170°C und einem konstanten Druck von 9 bar bis zur Beendigung der Wasserstoffbildung. Die freigesetzte Menge Wasserstoff wurde in Abhängigkeit der Reaktionszeit aufgezeichnet. Nach beendeter Reaktion wurde das Reaktionsprodukt analysiert. Die Ergebnisse sind in der nachfolgenden Tabelle zusammengefaßt:

**Tabelle**

| Katalysator | Reaktionszyklen | Reaktionszeit [min.] | Umsatz [%] | Selektivität [%] |
|---|---|---|---|---|
| Beispiel 3 | 1 | 164 | 99 | 98 |
| Beispiel 4 | 1 | 176 | 99 | 98 |
| Beispiel 5 | 1 | 108 | 99 | 98 |
| Vergleichsbeispiel A | 1 | 343 | 99 | 98 |
| Beispiel 3 | 10 | 212 | 99 | 95 |
| Beispiel 4 | 10 | 288 | 98 | 96 |
| Beispiel 5 | 10 | 281 | 99 | 96 |

## Patentansprüche

1. Verfahren zur Herstellung von Carbonsäuresalzen, **dadurch gekennzeichnet, daß** man Alkohole der allgemeinen Formel I
R-CH₂CH₂OH (I),
in der
R -(OCH₂CH₂OH) oder -NR¹R² und
R¹, R² unabhängig voneinander Wasserstoff, Phenyl, -CH₂CH₂OH, C₁- bis C₂₀-Alkyl oder C₂- bis C₅-Dialkylamino
bedeuten, mit Alkalihydroxid, Erdalkalihydroxid oder deren Gemischen in Wasser in Gegenwart eines Kupferkatalysators, der
a) aus Kupfer besteht oder
b) 99,9 bis 10 Gew.-% Kupfer und 0,01 bis 90 Gew.-% Eisen und 0 bis 50 Gew.-% eines oder mehrerer anderer Metalle enthält,
der gegebenenfalls dotiert ist, hergestellt durch Prezipitation von Kupfersalzlösungen bzw. von Kupfer- und Eisensalzlösungen ggf. mit Salzen anderer Metalle durch Coprezipitation mit einer Base und Reduktion mit Wasserstoff,
bei Temperaturen von 120 bis 280°C und einem Druck zwischen dem Dampfdruck des Wassers und 75 bar umsetzt.

2. Verfahren zur Herstellung von Carbonsäuresalzen nach Anspruch 1, **dadurch gekennzeichnet, daß** der Gehalt an Aluminiumionen im Reaktionsgemisch weniger als 4000 ppm beträgt.

## Claims

1. A process for preparing carboxylic acid salts, which comprises reacting alcohols of the general formula I
R-CH₂CH₂OH (I)
where
R is -(OCH₂CH₂OH) or -NR¹R² and
R¹ and R² are each independently hydrogen, phenyl, -CH₂CH₂OH, C₁- to C₂₀-alkyl or C₂- to C₅-dialkylamino
with an alkali metal hydroxide, alkaline earth metal hydroxide or a mixture thereof in water in the presence of a copper catalyst
which
a) consists of copper or
b) comprises from 99.9 to 10% by weight of copper and from 0.01 to 90% by weight of iron and from 0 to 50% by weight of one or more other metals,
and may, if appropriate, be doped, prepared by precipitation of copper salt solutions or by coprecipitation of copper and iron salt solutions optionally containing salts of other metals using a base, and reduction by hydrogen,
at temperatures of from 120 to 280°C and a pressure between the vapor pressure of the water and 75 bar.

2. The process for preparing carboxylic acid salts according to claim 1, wherein the content of aluminum ions in the reaction mixture is less than 4000 ppm.

## Revendications

1. Procédé de préparation de sels d'acide carboxylique, **caractérisé en ce qu'**on fait réagir, à des températures de 120 à 280°C et sous une pression entre la tension de vapeur de l'eau et 75 bars, des alcools de la formule générale I :
R-CH₂CH₂OH (I)
dans laquelle
R représente - (OCH₂CH₂OH) ou -NR¹R², et
R¹, R² représentent, indépendamment l'un de l'autre, de l'hydrogène ou un groupe phényle, -CH₂CH₂OH alkyle en C₁-C₂₀ ou dialkylamino en C₂-C₅,
avec de l'hydroxyde de métal alcalin, de l'hydroxyde de métal alcalino-terreux ou leurs mélanges, dans de l'eau, en présence d'un catalyseur à base de cuivre qui
a) est constitué de cuivre, ou
b) contient 99,9 à 10 % en poids de cuivre et 0,01 à 90 % en poids de fer et 0 à 50 % en poids d'un ou de plusieurs autres métaux,
est éventuellement dopé et est préparé par précipitation de solutions de sel de cuivre ou de solutions de sel de cuivre et de fer, éventuellement avec des sels d'autres métaux, par coprécipitation avec une base et réduction avec de l'hydrogène.

2. Procédé de préparation de sels d'acide carboxylique suivant la revendication 1, **caractérisé en ce que** la teneur en ions aluminium dans le mélange réactionnel est inférieure à 4 000 ppm.
